# EUROPÄISCHE PATENTANMELDUNG

(11) **EP 3 572 104 A1**
(43) Veröffentlichungstag der Anmeldung: **27.11.2019**
(21) Anmeldenummer: 18174341.0
(22) Anmeldetag: 25.05.2018
(51) Int. Cl.: A61M 1/10, A61M 1/12

(54) **BAUTEIL ZUM FÜHREN EINES FLUIDS MIT EINEM SENSOR**

(71) Anmelder: Berlin Heart GmbH, 12247 Berlin (DE)
(72) Erfinder: PHILLIPS, Daniel, 10249 Berlin (DE); DR. FRISCHKE, Michael, 15834 Rangsdorf (DE); PETERS, Oliver, 14129 Berlin (DE)
(74) Vertreter: Pfenning, Meinig & Partner mbB

(57) **Zusammenfassung**

Die vorliegende Erfindung betrifft ein Bauteil zum Führen eines Fluids mit einem Sensor, wobei das Bauteil eine innere und eine äußere Wandung umfasst, wobei die innere Wandung zum Führen des Fluids ausgebildet ist, die äußere Wandung das Bauteil nach außen abschließt und zwischen der inneren und der äußeren Wandung ein Wandbereich ausgebildet ist. Das erfindungsgemäße Bauteil zeichnet sich dadurch aus, dass der Sensor ein elektromechanisches Sensorelement aufweist und im Wandbereich an der inneren Wandung angeordnet ist, wobei der Sensor eingerichtet ist, mittels des Sensorelements einen Grad einer Verformung der inneren Wandung im Bereich des Sensors zu messen und als elektrisches Signal auszugeben, wobei das elektromechanische Sensorelement eine Länge und/oder eine Breite von ≤ 50 µm aufweist.

## Beschreibung

Die vorliegende Erfindung betrifft ein Bauteil zum Führen eines Fluids mit einem Sensor zur Messung einer Verformung des Bauteils. Weiterhin betrifft die Erfindung ein Verfahren zum Herstellen des Bauteils enthaltend den Sensor. Das betreffende Bauteil kann insbesondere im Bereich von Herzunterstützungssystemen eingesetzt werden.

Mit dem heutigen Stand der Technik im Bereich der Herzunterstützungssysteme sind die Anforderungen an die zu integrierende Sensorik gestiegen, die in modernen Herzunterstützungssystemen zunehmend an Bedeutung gewinnen, um einen Patienten sicher und gemäß des individuellen Support-Bedarfs versorgen und unterstützen zu können. Gemeint sind primär jene Anforderungen, die eine blutverträgliche und gleichzeitig zuverlässige Messung verschiedener Kenngrößen zulassen (z.B. Druckmessung, Flussmessung, Materialermüdung, Vibrationsmessung, Anschlagdetektion, Detektion eines Thrombendurchgangs, Detektion und Unterscheidung systematischer und zufälliger Störgrößen).

Gegenwärtig können viele für den Anwender aus technischer als auch klinischer Sicht wichtige Eigenschaften und Zustände eines Herzunterstützungssystems bzw. Patienten aufgrund der verfügbaren Technik nicht erfasst und verarbeitet werden. Das ist zum einen bedingt durch in den Herzunterstützungssystemen angewandte Sensor-Technologie, als auch durch den oftmals kaum verfügbaren Bauraum, der für bestimmte Messgrößen und an verschiedenen Stellen innerhalb eines Herzunterstützungssystems eine sogar oftmals mehrfache Applikation diverser Sensoren erfordert. Des Weiteren müssen häufig strömungstechnische Einbußen oder Einbußen hinsichtlich der hämokompatiblen Blutführung in Kauf genommen werden, da beispielsweise das Einschweißen einer flachen Membran als Aufnahme für die Sensoren erforderlich ist (z.B. Einschränkung der strömungstechnischen Gestaltung, Aktivierung durch Rauheiten einer Schweißnaht/Spalt/Stufe, etc.), oder die geometrische Beschaffenheit der blutführenden Strecke für die Anwendbarkeit der jeweiligen Messapparatur entsprechend angepasst werden muss (z.B. Abflachung der Innenwand, großflächiges ausdünnen einer Rohrwand oder Tangentialkammer, Ausbeulen einer blutführenden Wandung um Bauraum zu schaffen).

Zudem muss jedwede Sensorik in dem Einlassbereich oder Auslassbereich einer Blutpumpe elektrisch an das Gesamtsystem angebunden werden. Dazu zählt auch das elektrische Kontaktieren von einzelnen physikalischen Messaufnehmern oder das Verbinden ganzer Baugruppen. Aufgrund enger Platzverhältnisse zwischen Innenwand und Außenwand der Blutpumpe müssen die Sensorelemente und auch die Verdrahtung eine geringe Bauhöhe aufweisen.

Die Aufgabe der vorliegenden Erfindung ist es, ein Bauteil zum Führen eines Fluids mit einem Sensor, beispielsweise für ein Herzunterstützungssystem, bereitzustellen, welches die oben beschriebenen Probleme löst und wobei der Sensor in das Bauteil derart integriert ist, dass wichtige Zustände eines mit dem Bauteil verbindbaren Fluidsystems, beispielsweise eines Herzunterstützungssystems als auch eines mit dem Herzunterstützungssystem in Wechselwirkung stehenden Patienten, erfasst werden können, die Ausmaße des Bauteils nicht vergrößert werden, der Sensor das Fluid bzw. den Fluidfluss nicht beeinflusst und nicht mit einer Einbauumgebung des Bauteils in Kontakt kommt.

Die Aufgabe wird gelöst durch ein Bauteil zum Führen eines Fluids mit einem Sensor nach Anspruch 1 sowie durch ein Verfahren zum Herstellen eines derartigen Bauteils nach Anspruch 12. Vorteilhafte Weiterbildungen des erfindungsgemäßen Bauteils sind in den abhängigen Ansprüchen 2 bis 11 und 13 bis 15 aufgeführt.

Das erfindungsgemäße Bauteil zum Führen eines Fluids mit einem Sensor umfasst eine innere und eine äußere Wandung, wobei die innere Wandung zum Führen des Fluids ausgebildet ist, die äußere Wandung das Bauteil nach außen abschließt und zwischen der inneren und der äußeren Wandung ein Wandbereich ausgebildet ist. Das erfindungsgemäße Bauteil zeichnet sich dadurch aus, dass der Sensor ein elektromechanisches Sensorelement aufweist und im Wandbereich an der inneren Wandung angeordnet ist, wobei der Sensor eingerichtet ist, mittels des Sensorelements einen Grad einer Verformung der inneren Wandung im Bereich des Sensors zu messen und als elektrisches Signal auszugeben, wobei das elektromechanische Sensorelement eine Länge und/oder eine Breite von ≤ 50 µm aufweist.

Unter einem Sensor wird hier ein Schichtaufbau verstanden, der vorzugsweise in einer ersten Schicht zwei elektrische Kontakte aufweist, die durch einen Spalt voneinander separiert sind. Auf den elektrischen Kontakten kann dann das Sensorelement in einer zweiten Schicht angeordnet sein. Das Sensorelement ist ein elektromechanisches Element, vorzugsweise ein nanostrukturelles Dehnelement, und weist selbst keine weiteren Bauteile, wie z.B. elektrische Leiter, auf. Das Sensorelement erstreckt sich vorzugsweise in einer Querrichtung über den Spalt. Weiterhin kann das Sensorelement im Bereich des Spalts in die erste Schicht hineinragen. Weiterhin umfasst der Sensor vorzugsweise zwei Bond-Pads, wobei jeweils ein Bond-Pad mit einem der elektrischen Kontakte elektrisch kontaktiert ist.

Die innere Wandung weist auf der Fluid-führenden Seite auch nach Einbetten des Sensors keine Sensor-bedingten Materialübergänge, Stufen oder Spalte (wie z.B. Schweißnähte o.Ä.) auf.

Die hier beschriebene Erfindung ist für alle implantierbaren und hämokompatiblen Werkstoffe anwendbar, und ist in der Lage kleinste Auslenkungen (Hübe, Vibrationen, Impulse) ohne invasive Einwirkungen auf ein blutführendes Element zu messen, die mit herkömmlichen Sensoren nicht erfasst werden können (z.B. DMS, Ultraschallsensoren, optische Sensoren), und diese Information auszugeben. Im gleichen Maße verhält es sich mit dem erforderlichen Bauraum, da der angewandte Sensor selbst nur wenige Kubikmikrometer in Anspruch nimmt, somit sehr leicht zu integrieren, und zum Teil in bislang unzugänglichen Strukturen applizierbar ist.

In einer vorteilhaften Ausgestaltung der Erfindung kann das Sensorelement ein nanostrukturelles Dehnelement sein, welches stoffschlüssig auf die Kontakte aufgebracht ist, sodass die Kontakte über das Sensorelement elektrisch miteinander kontaktiert sind, wobei die elektrischen Kontakte derart auf die innere Wandung aufgebracht sind, dass sich das Sensorelement bei Verformung der inneren Wandung im Bereich des Sensors mit der inneren Wandung verformt, sodass zwischen den elektrischen Kontakten das elektrische Signal messbar ist.

Weiterhin kann im Wandbereich an der inneren Wandung eine Vielzahl von Sensoren angeordnet sein. Insbesondere können an der inneren Wandung vier Sensoren angeordnet sein, welche vorzugsweise zu einer Voll- oder Halbbrücken-Schaltung miteinander verschaltet sind, um eine Temperaturabhängigkeit der Sensoren zu reduzieren.

Weiterhin kann zwischen der inneren Wandung und den elektrischen Kontakten des Sensors eine elektrische Isolationsschicht angeordnet sein, welche stoffschlüssig mit der inneren Wandung und den elektrischen Kontakten verbunden ist.

Das Sensorelement kann insbesondere ein nanokristallines Verbundmaterial (Nanocomposite) aufweisen, welches vorzugsweise mittels Nano-3D-Drucks aufdruckbar ist und dessen Komposition beliebig veränderbar ist. Weiterhin kann das Sensorelement ein Metall und/oder ein Halbleitermaterial aufweisen. Das Sensorelement kann auch ein mit Ladungsträgern dotiertes Metall und/oder ein mit Ladungsträgern dotiertes Halbleitermaterial aufweisen, um eine elektrische Leitfähigkeit und/oder einen elektrischen Widerstand des Sensorelements einzustellen.

Das elektrische Signal kann insbesondere eine Änderung eines elektrischen Widerstands des Sensors beinhalten.

Vorteilhaft ist es, wenn das Sensorelement eine Länge und/oder eine Breite von ≤ 15µm, insbesondere ≤ 10 µm, insbesondere ≤ 3 µm und/oder eine Dicke von ≤ 50 µm, insbesondere ≤ 15µm, insbesondere ≤ 10 µm, insbesondere ≤ 3 µm aufweist.

Vorzugsweise können die elektrischen Kontakte derart auf die innere Wandung aufgebracht sein, dass zwischen den Kontakten ein Spalt von ≤ 50 µm, insbesondere ≤ 30 µm, insbesondere ≤ 15µm, insbesondere ≤ 10 µm, insbesondere ≤ 3 µm besteht, wobei der Spalt in einer Querrichtung vollständig von dem Sensorelement überdeckt wird.

Weiterhin ist es bevorzugt, wenn der Sensor in einem abgedünnten Bereich der inneren Wandung angeordnet ist. Eine Wandstärke in dem abgedünnten Bereich ist vorzugsweise ≥ 5 µm, insbesondere ≥ 100 µm und/oder ≤ 500 µm, insbesondere ≤ 200 µm.

Das Bauteil kann insbesondere eine Blutpumpe sein, wobei ein oder mehrere Sensoren im Einlassbereich und/oder im Auslassbereich im Wandbereich an der inneren Wandung der Blutpumpe angeordnet sind, und aus der Verformung der inneren Wandung ein Blutdruck im Einlassbereich und/oder Auslassbereich, eine momentane Durchflussmenge und/oder Laufeigenschaften der Blutpumpe bestimmbar sind.

Das Bauteil kann weiterhin eine Blutpumpe mit einem beweglichen Förderelement sein, wobei ein oder mehrere Sensoren an der inneren Wandung im Wandbereich der Blutpumpe angeordnet sind, und aus der Verformung der inneren Wandung ein Anschlag des Förderelements an der inneren Wandung festellbar ist.

Das Bauteil kann weiterhin eine mechanisch gelagerte Rotationsblutpumpe ist, wobei ein oder mehrere Sensoren im Bereich der Lageraufnahme im Wandbereich an der inneren Wandung angeordnet sind, und aus der Verformung der inneren Wandung eine Lagerkraft und/oder ein Lagerverschleiß bestimmbar ist.

Weiterhin kann eine Vielzahl von Sensoren entlang eines Umfangs der inneren Wandung im Wandbereich oder in Bereich der inneren Wandung ähnlicher statischer Druckverhältnisse angeordnet sein. Mithilfe der Vielzahl an Sensoren können Mittelwerte der Messwerte gebildet und so Messungen der Sensoren genauer gestaltet werden.

Gemäß einer weiteren Ausgestaltung der Erfindung können in dem Wandbereich elektrische Verbindungselemente angeordnet sein, über welche der Sensor mit einer Verarbeitungseinrichtung verbunden ist.

Insbesondere können die Verbindungselemente eine Leiterplatte, wobei ein oder mehrere Sensoren direkt über die elektrischen Kontakte oder über Bonddrähte mit der Leiterplatte verbunden sind, oder einzeln isolierte Leitungen, insbesondere Teflon-isolierte Leitungen, umfassen.

Weiterhin können auf der Leiterplatte elektrische Bauelemente zur Vorverarbeitung der elektrischen Signale, insbesondere zur Vorverstärkung angeordnet sein.

Die vorliegende Erfindung umfasst auch ein Verfahren zur Herstellung eines Bauteils zum Führen eines Fluids mit einem Sensor. Das erfindungsgemäße Verfahren umfasst die folgenden Schritte: Ausbilden einer inneren und einer äußeren Wandung des Bauteils, wobei die innere Wandung zum Führen des Fluids ausgebildet ist und die äußere Wandung ausgebildet ist, das Bauteil nach außen abzuschließen, und die innere und die äußere Wandung derart zusammenfügbar sind, dass sich zwischen der inneren und der äußeren Wandung ein Wandbereich ergibt, Anordnen eines Sensors mit einem elektromechanischen Sensorelement im Wandbereich an der inneren Wandung, wobei der Sensor eingerichtet ist, mittels des Sensorelements einen Grad einer Verformung der inneren Wandung im Bereich des Sensors zu messen und als elektrisches Signal auszugeben, wobei das elektromechanische Sensorelement eine Länge und/oder eine Breite von ≤ 50 µm aufweist, und Zusammenfügen der inneren und äußeren Wandung.

In einer weiteren vorteilhaften Ausgestaltung des erfindungsgemäßen Verfahrens kann vor dem Anordnen des Sensors eine Isolationsschicht im Wandbereich auf die innere Wandung aufgebracht werden. Dies ist insbesondere notwendig, wenn die innere Wandung ein elektrisch leitendes Material aufweist, um einen Kurzschluss zwischen den Kontakten zu verhindern.

Das Anordnen des Sensors kann insbesondere die folgenden Schritte umfassen: Aufbringen von elektrischen Kontakten auf der inneren Wandung bzw. auf der isolationsschicht zum Abnehmen des elektrischen Signals und Aufbringen eines elektromechanischen Sensorelements auf den elektrischen Kontakten.

Insbesondere kann das Verfahren umfassen, dass vor dem Anordnen des Sensors an der inneren Wandung oder vor dem Aufbringen der Isolationsschicht eine Wandstärke der Wandung im Bereich des Sensors abgedünnt wird, insbesondere auf eine Wandstärke von ≥ 5 µm, insbesondere ≥ 100 µm und/oder ≤ 500 µm, insbesondere ≤ 200 µm abgedünnt wird.

Weiterhin kann das Verfahren umfassen, dass das Sensorelement mittels Nano-3D-Drucks, Aufsputterns oder mittels eines Ätzverfahrens auf die Isolationsschicht aufgebracht wird.

Weiterhin kann das Verfahren umfassen, dass eine Empfindlichkeit des Sensorelements durch Einbringen einer höheren oder geringeren Anzahl von Ladungsträgern in das Sensorelement eingestellt wird.

Im Folgenden werden verschiedene Ausführungsbeispiele eines erfindungsgemäßen Bauteils anhand von Figuren detaillierter beschrieben. Dabei werden verschiedene erfindungswesentliche oder auch vorteilhafte weiterbildende Elemente im Rahmen jeweils eines konkreten Beispiels genannt, wobei auch einzelne dieser Elemente als solche zur Weiterbildung der Erfindung - auch herausgelöst aus dem Kontext des jeweiligen Beispiels und weiterer Merkmale des jeweiligen Beispiels - verwendet werden können. Weiterhin werden in den Figuren für gleiche oder ähnliche Elemente gleiche oder ähnliche Bezugszeichen verwendet, und deren Erläuterung daher teilweise weggelassen.

Es zeigen
- Figur 1: a) eine Draufsicht auf einen Teil eines Sensors, b) - c) eine Sensorapplikation an verschiedenen Varianten einer inneren Wandung unterschiedlicher Geometrien,
- Figur 2: verschiedene Varianten eines Blutpumpenrohres mit Sensoren,
- Figur 3: a) - g) verschiedene Varianten zur elektrischen Anbindung eines Sensors in einem Blutpumpenrohr,
- Figur 4: eine vereinfachte Darstellung der Anbindung der Sensoren an eine Verarbeitungseinrichtung,
- Figur 5: a) - f) verschiedene Varianten von Signalketten von einem oder mehreren Sensoren zu einem Kommunikationsmodul und
- Figur 6: a) Draufsicht auf einen Sensorbereich mit sechs Sensorstrukturen, b) schematische Darstellung einer Sensorstruktur bestehend aus vier zu einer Vollbrücke verschalteten Sensoren.

Die folgenden Ausführungsbeispiele beziehen sich vorwiegend auf das Gebiet der Blut-führenden Bauteile, wie z.B. der Blutpumpen für Herzunterstützungssysteme. Die Erfindung beschränkt sich jedoch nicht auf dieses Gebiet, sondern ist auf Fluid-führende Bauteile jeglicher Art anwendbar.

Ein Ziel der vorliegenden Erfindung in Bezug auf Blut-führende Bauteile ist es, kleinste Auslenkungen (Hübe, Vibrationen, Impulse) ohne invasive Einwirkungen auf ein Blut-führendes Element zu messen, und diese Information auszugeben. Hierzu können spezifische Messkonfigurationen bereitgestellt werden, welche die Messung von Signalen ohne negativen Einfluss auf strömungstechnische als auch hämokompatible Performance eines blutführenden und oder blutfördernden Systems ermöglichen.

Dieses Ziel wird erreicht, indem eine oder mehrere Nano-, bzw. Mikrostrukturen (Sensorelement) in einem entsprechenden Abstand und Verlauf auf einen hämokompatiblen, oder beschichteten Werkstoff (Bauteil) aufgebracht werden. Da diese Nanostrukturen als Leiterbahnen dienen, bestehen sie aus einem leitfähigem Material welches mit Hilfe von mikrosytemtechnischen Fertigungsverfahren aufgebracht werden kann (z.B. Lithografisch, Mikroätzen, PEVDM, etc.). Eine weitere Voraussetzung ist die elektronisch passive Applikationsfläche, auf der das Sensorelement appliziert wird, was im Falle einer leitfähigen inneren Wandung zusätzlich eine Isolationsschicht erfordert. Zwischen zwei Leiterbahnen (elektrischen Kontakten) wird dann ein Nanostrukturelles Dehnelement aufgebracht, das bei kleinsten Auslenkungen aufgrund der daraus resultierenden Längenänderung des Dehnelements zu einer veränderten Leitfähigkeit im Dehnelement führt, und somit ein Spannungsdelta zwischen den Leiterbahnen messbar macht.

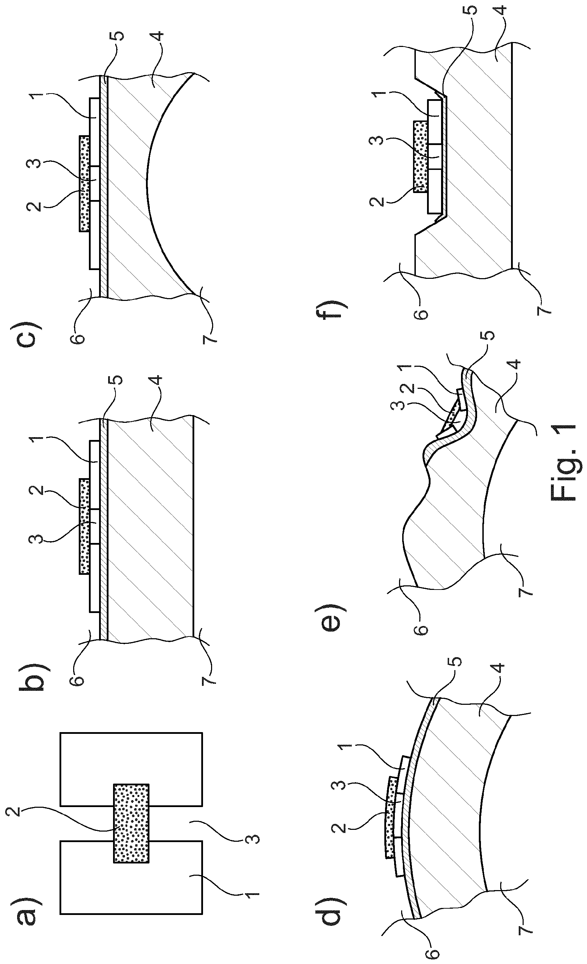

Figur 1a) zeigt eine schematische (nicht maßstabsgetreue) Draufsicht auf einen Teil eines einzelnen Sensors. Zu sehen sind zwei flächig ausgebildete elektrische Kontakte 1, welche so nebeneinander angeordnet sind, dass sich ein Spalt 3 von etwa ≤ 50 µm zwischen den Kontakten 1 ausbildet. Der Spalt 3 wird in einer Querrichtung von einem nanostrukturellen Dehnelement 2 als Sensorelement überdeckt. Hier nicht dargestellt sind zwei Bond-Pads des Sensors, wobei je eine Kontaktfläche 1 mit je einem Bond-Pad zur Anbindung des Sensors an eine Verarbeitungseinrichtung kontaktiert ist.

Figuren 1 b) - f) zeigen eine Anordnung eines Sensors an verschiedenen Beispielen einer inneren Wandung mit unterschiedlichen Geometrien. Die Gestalt der direkten Applikationsfläche der inneren Wandung, auf welcher der Sensor angeordnet wird, spielt hier eine untergeordnete Rolle, da lediglich eine hinreichende Auslenkung erforderlich ist, die im Nano- bis Subnanometerbereich liegen kann. Zur Quantifizierung der zu erwartenden Auslenkung können eine Vielzahl von Messtechniken oder auch FEM-Simulationen dienlich sein. Auch Freiformflächen als Applikationsflächen sind möglich. Figur 1 b) zeigt einen Querschnitt durch eine ebene innere Wandung 4 in einem Sensorbereich. Die innere Wandung 3 ist sowohl auf einer Wandbereichsseite 6 als auch auf einer Fluidbereichsseite 7 eben ausgebildet. Auf der inneren Wandung 4 im Wandbereich ist eine Isolationsschicht 5 aufgebracht. Auf der Isolationsschicht 5 sind die elektrischen Kontaktflächen 1 unter Ausbildung eines Spalts 5 aufgebracht. Auf den Kontaktflächen 1 ist den Spalt 3 überbrückend das Sensorelement 2 angeordnet. Figur 1 c) zeigt einen Querschnitt durch einen Sensorbereich einer inneren Wandung 4. Auf der Wandbereichsseite 6 ist die innere Wandung 4 eben ausgebildet, auf eine Fluidbereichsseite 7 ist die innere Wandung 4 konkav geformt. Zur Optimierung des Sensorsignals ist der Sensor an einer Stelle minimaler Wandstärke der inneren Wandung angeordnet. Figur 1 d) zeigt einen Querschnitt durch einen Sensorbereich einer inneren Wandung 4, welche insgesamt gekrümmt ist, sodass ihre Wandstärke konstant ist. Figur 1 e) zeigt einen Querschnitt durch einen Sensorbereich einer inneren Wandung 4, wobei die Wandbereichsseite 6 eine komplizierte Geometrie aufweist und die Applikationsfläche des Sensors eine Freiformfläche darstellt. Die Fluidbereichsseite 7 ist konkav geformt, und der Sensor ist vorteilhafterweise an einer Stelle minimaler Wandstärke der inneren Wandung 4 angeordnet zur Optimierung des Sensorsignals. Ebenso ist eine Sensorapplikation in einem Bereich der inneren Wandung 4 möglich, in welchem sowohl die Wandbereichsseite 6 als auch die Fluidbereichsseite 7 komplizierte Geometrien und Freiformflächen aufweisen. Figur 1 f) zeigt einen Querschnitt durch einen Sensorbereich einer inneren Wandung 4, wobei die innere Wandung im Sensorbereich beispielsweise mittels Einfräsens, Bohrens oder eines chemischen Prozesses abgedünnt wurde. Bei einer nicht-leitenden inneren Wandung kann auf eine Isolationsschicht verzichtet werden. Wie aus den Figuren 1 c) - f) ersichtlich ist, weist die innere Wandung 4 auf der Fluid-führenden Seite 7 auch nach Einbetten des Sensors keine Sensor-bedingten Materialübergänge, Stufen oder Spalte (wie z.B. Schweißnähte o.Ä.) auf.

Folgende Integrationsmöglichkeiten für Sensoren innerhalb eines Blutfördernden oder Blut-führenden Systems sind möglich:

### 1. Druckmessung in einem Blutpumpenrohr

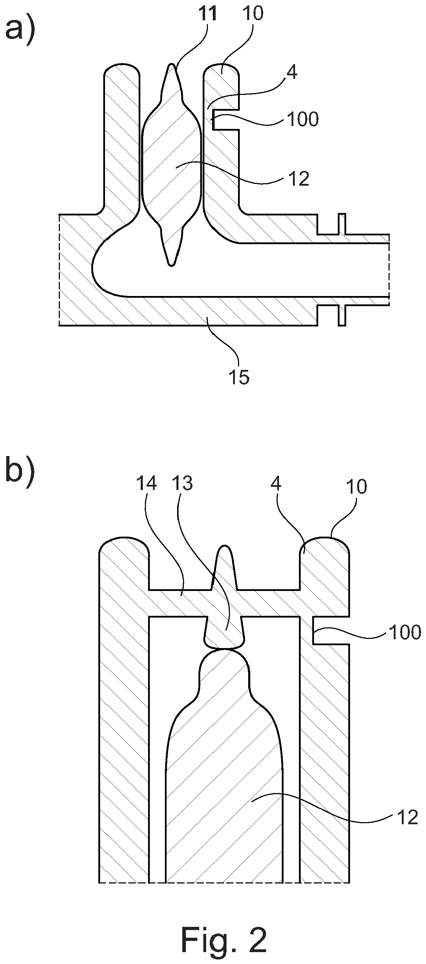

Figur 2 a) zeigt eine innere Wandung 4 eines Blutpumpenrohrs 10 mit einer darin angeordneten Axialfluss-Blutpumpe 11. Zur Messung eines Blutdrucks in einem mit dem Blutpumpenrohr 10 verbundenen Ventrikels werden an einer abgedünnten Stelle der inneren Wandung 4 im Einlassbereich (ggf. zusätzlich im Bereich der Auslasskanüle/Diffusors) des Blutpumpenrohrs 10 ein oder mehrere Sensoren 100 in einer in Figuren 1 c) - f) beschriebenen Konfiguration appliziert. Die gemessene Auslenkung lässt auf den Ventrikeldruck schließen, wenn der Normaldruck oder z.B. der Druck innerhalb des hermetisch dichten Blutpumpenrohres 10 als Referenz zur Verfügung steht. Der Druck innerhalb des hermetisch dichten Blutpumpenrohres 10 kann mithilfe eines im Blutpumpenrohr 10 angeordneten Referenzdrucksensors (z.B. eines Absolutdrucksensors) ermittelt werden.

### 2. Flussmessung

Zur Bestimmung der momentanen Durchflussmenge in einer Blutpumpe werden im Einlass-, und Auslassbereich eines Blutpumpenrohres 10 eine oder mehrere Sensoren 100 in einer in Figuren 1 c) - f) beschriebenen Konfiguration appliziert. Mithilfe einer HQ-Kennlinie des jeweiligen Herzunterstützungssystems kann der momentane Blutfluss bestimmt werden, da der Ventrikeldruck (Anwendung 1.) bekannt ist.

### 3. Anschlagdetektion

Zur Erkennung eines Anschlagevents in einem Herzunterstützungssystem, welches einen Rotor 12 oder ein bewegliches Element zur Förderung/Führung von Blut aufweist, werden ein oder mehrere Sensoren 100 in einer wie in den Figuren 1 c) - f) und Figur 2 a) und/oder b) appliziert. In Figur 2 b) ist der Sensor 100 nahe einer Lageraufnahme 13 für den Rotor 12 angeordnet. Alternativ oder zusätzlich zu den in Figuren 2 a) und b) gezeigten Applikationsmöglichkeiten für die Sensoren 100 kann eine Vielzahl von Sensoren 100 auch gleichmäßig über die gesamte innere Wandung 4 des Blutpumpenrohres 10 verteilt angeordnet sein, in einer Einlass- und/oder Auslasskanüle (Silikonkanüle, Kunststoffrohr, Metallisches Rohr) und/oder in einem Konnektor am Einlass/Auslass (Metall, Kunststoff) eingebettet sein. Aus den während des Events auftretenden Auslenkimpulsen an der oder den Sensorflächen, kann auf eine ungewollte Anschlagsituation geschlossen werden, und entsprechende Maßnahmen oder Signalisierung eingeleitet werden.

### 4. Messung der Lagerkräfte

Ein Rotor 12 innerhalb eines mechanisch gelagerten Herzunterstützungssystems erzeugt beim Fördern von Blut in Abhängigkeit des momentanen Gegendrucks eine axiale Kraft, die in einem mechanischen Lager 13 aufgenommen wird. Zur Bestimmung der Lagerkraft werden ein oder mehrere Sensoren 100 in der in Figur 2 b) dargestellten Konfiguration appliziert. Die sich im Einlass befindende Lageraufnahme 13 ist dabei derart gestaltet, das die aufgenommene Lagerkraft (Axialkraft des Rotors 12) zu einer Auslenkung führt, die entweder direkt an einer Strebe 14 oder Blutrohrwand 4 gemessen wird. Mithilfe der HQ-Kennlinie des jeweiligen Herzunterstützungssystems kann der momentane Blutfluss bestimmt werden, da der Ventrikeldruck (Anwendung 1) bekannt ist.

### 5. Verschleiß an mechanischen Lagern

Ein Rotor 12 innerhalb eines mechanisch gelagerten Herzunterstützungssystems erzeugt beim Fördern eine axiale Kraft, die in einem mechanischen Lager 13 aufgenommen wirkt. Wird dieser Rotor 12 mit einer bestimmten und als bekannt vorausgesetzten Vorspannung eingebaut (ist üblicherweise erforderlich, um eine Spaltbildung zwischen den Lagerflächen zu vermeiden), ist eine Auslenkung im Lager 13 zu messen (Anwendung 4). Wenn durch Abrieb im Lager 13 die axiale Ausdehnung des Rotors 12 reduziert wird, sinkt dadurch auch die durch die Vorspannung erzeugte Auslenkung am Lagerpunkt 13. Durch die hochauflösende Messung der Sensoren 100 kann somit der Verschleiß an einem Lager 13 detektiert, und entsprechende Maßnahmen eingeleitet werden, falls eine gefährdende Situation vorliegt.

### 6. Materialermüdung

Die in Anwendung 5 beschriebenen Störgrößen lassen gleichermaßen auf eine Materialermüdung am Lager 13 schließen.

### 7. Vibrationserkennung

Da die Sensoren 100 kleinste Auslenkungen erfassen, sind sie auch zur Überwachung der Laufeigenschaften eines Herzunterstützungssystems geeignet (Messung periodischer Schwingungen). Ungewollte Vibrationen oder sporadische Impulse, die z.B. beim Fallen eines Patienten, oder durch eine starke Verzögerung bei z.B. einem Unfall auftreten, können daher erkannt und entsprechend verarbeitet werden.

### 8. Erkennung/Unterscheidung sporadischer Störgrößen und systematischer Störgrößen

Logische Verarbeitung der in Anwendung 7 gemessenen Signale.

### 9. Thrombendetektion

Bei einem Thrombendurchgang in einer Blutpumpe sind verschiedene Szenarien denkbar, die zu einer mechanischen Schwingung/Impuls führen. Mit einer schwingungssensitiven Blutpumpe könnte daher ein solches Event erkannt und zur Einleitung entsprechender Maßnahmen verwendet werden.

Zum Betrieb des Sensors 100 im Blutpumpenrohr 10 oder in der Auslasskammer/Auslassrohr 15 ist eine elektrische Anbindung an eine Verarbeitungseinrichtung notwendig. Die elektrische Anbindung kann über zwischen der äußeren Wandung und der inneren Wandung angeordnete elektrische Verbindungselemente erfolgen. Implantierbare Rotationsblutpumpen bestehen i.d.R. aus einem inneren Pumpenrohr als innerer Wandung, dessen innere Oberfläche das Blut führt. Außerhalb des inneren Pumpenrohrs sind bei fast allen Blutpumpen der Motor oder andere elektronische bzw. elektromechanische Baugruppen untergebracht, z.B. Sensoren, aktive Magnetlagerkomponenten. Teile dieser Sensoren können auch als ungekapselte Messaufnehmer implementiert sein. Zur hermetischen Abdeckung dieser Komponenten wird ebenfalls bei fast allen Rotationsblutpumpen ein äußeres Pumpenrohr bzw. ein Gehäuse als äußere Wandung aufgeschweißt. Innerhalb des Hohlraums (Wandbereich) zwischen innerem und äußerem Pumpenrohr bzw. Gehäuse befinden sich die elektrischen Verbindungselemente.

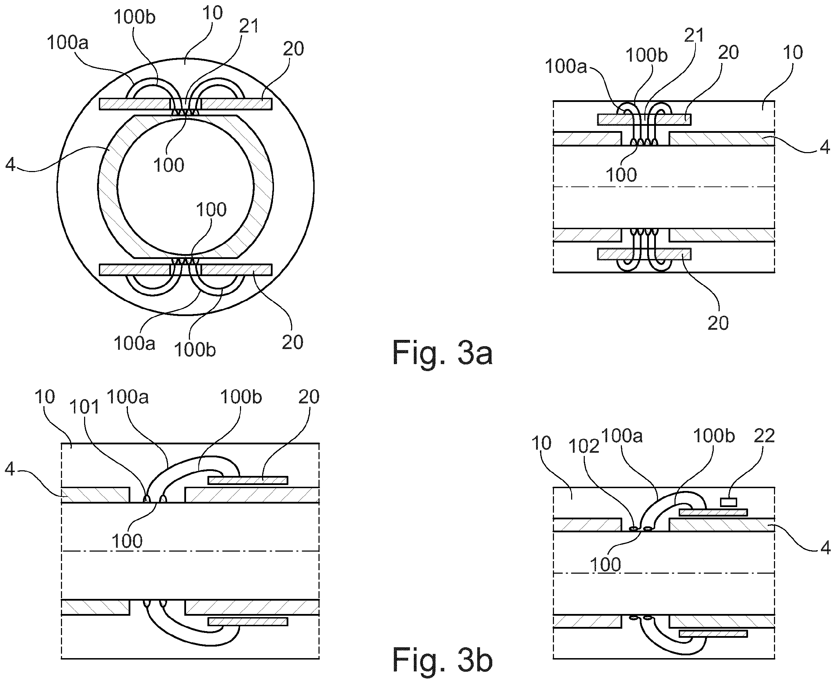

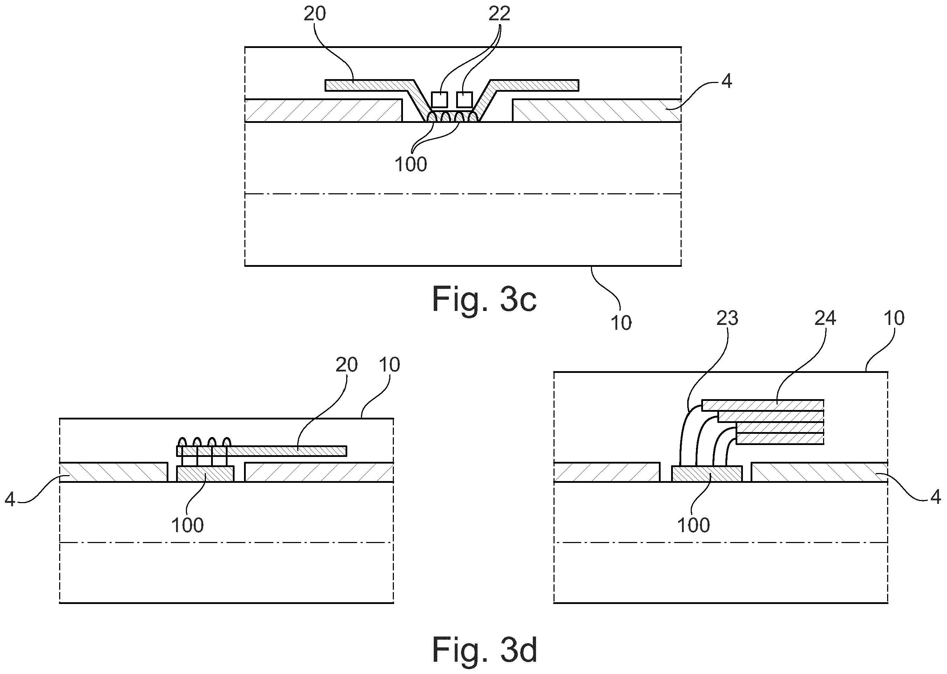

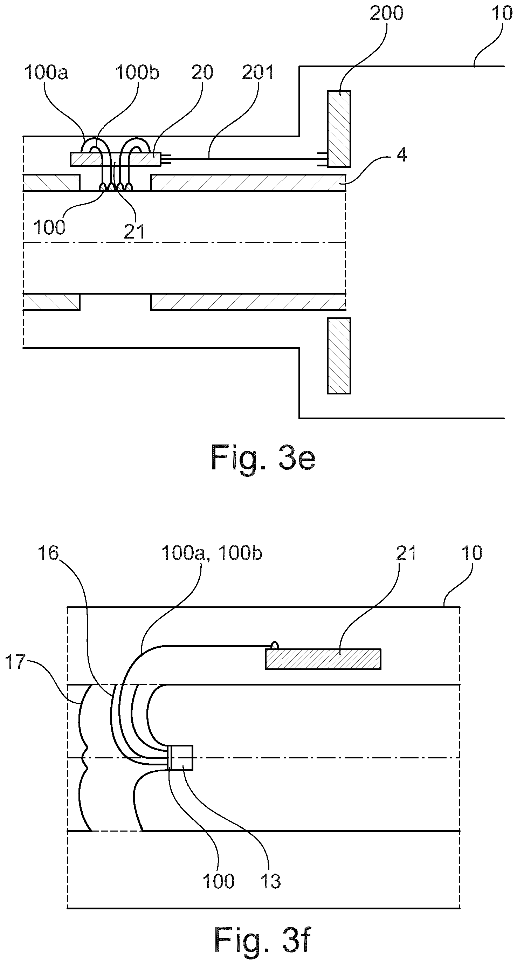

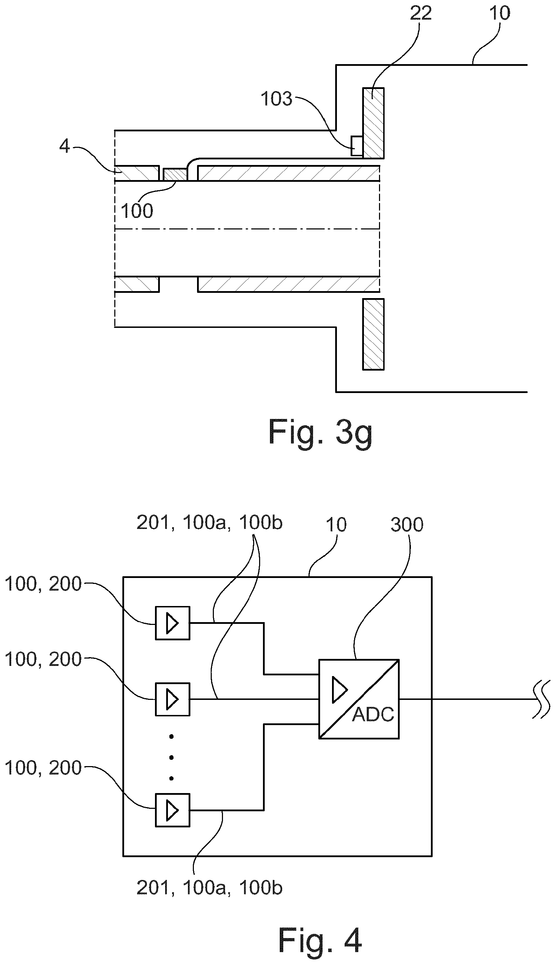

In den Figuren 3 a) - g) werden Verbindungsstrukturen gezeigt, die es ermöglichen, die Sensoren 100 elektrisch anzubinden. Es soll auch möglich sein, jeweils mehr als einen Sensor 100 zu konnektieren. Die Anzahl liegt sinnvollerweise zwischen einem und vier Sensoren. Zusätzlich ist die elektrische Anbindung und eine Signal-Vorverarbeitungseinrichtung dargestellt.

Figur 3 a) zeigt links einen Querschnitt und rechts einen Längsschnitt durch ein Pumpenrohr 10. Eine Leiterplatte (PCB) oder MID nimmt die zwei Bonddrähte 100a und 100b jeweils eines Sensors 100 auf, der auf dem Innerohr 4 des Pumpenrohrs 10 platziert ist. Die Bonddrähte 100a, 100b sind als Ball mittels Drahtbonden aufgebracht und werden auf der Leiterplatte 20 umverdrahtet. Die (flexible) Leiterplatte 20 liegt direkt über dem Sensor 100 und weist ein Loch oder eine Durchführung 21 für die Bonddrähte 100a, 100b auf.

Figur 3 b) zeigt eine Leiterplatte (PCB) oder MID 20 neben dem Sensor 100. Die Bonddrähte werden als Ball 101 (links) oder Wedge 102 (rechts) an dem Sensor 100 angebracht und auf der Platine umverdrahtet. Zusätzlich können auf der Leiterplatte 20 bereits passive oder aktive elektrische Bauelemente 22 als Vorverarbeitungseinrichtung untergebracht sein, um eine erste Filterung oder Vorverarbeitung durchzuführen.

Figur 3 c) zeigt eine 3D-MID Leiterplatte 20 als Verbinderplatine. Diese benötigt keine Bonddrähte zur Anbindung und kann direkt auf den Sensor 100 gelötet oder gesteckt werden. Zusätzlich können hier bereits elektrische Bauelemente 22 als Vorverarbeitungseinrichtung vorgesehen werden.

Figur 3 d) zeigt eine komplette Sensorbaugruppe 100, die entweder mit einer aufgesteckten und angelöteten Platine 20 oder über einzeln isolierte Adern 23 elektrisch verbunden wird. Die Isolation 24 kann beispielsweise über einen Teflonmantel erfolgen, der eine extrem hohe Spannungsfestigkeit bei sehr geringer Dicke aufweist.

In Figur 3 e) wird die elektrische Verbindung einer Umverdrahtungsplatine 20 am Sensor 100 zu einer Hauptplatine oder weiteren Pumpenelektronik 200 in dem Pumpenrohr 10 dargestellt. Diese Verbindung wird durch angesteckte oder aufgelötete galvanische Leiter 201 hergestellt und übermittelt die Sensordaten zur Weiterarbeitung an die Pumpenelektronik 200. Die Weiterleitung kann auch über 3D-geformte Platinen (z.B. MID Platinen) durchgeführt werden. Eine weitere Möglichkeit, die Daten innerhalb des Pumpenrohrs 10 zu übertragen ist eine drahtlose Kommunikationsschnittstelle.

In Figur 3 f) weist das Pumpenrohr 10 einen Lagerstator 13 auf, der in der Strömung liegt. Ein Sensor 100 in diesem Lagerstator 13 wird über einen Kanal 16 innerhalb der Statorstruktur 17 elektrisch verbunden. Hierzu werden Leitungen 100a, 100b durch den Kanal 16 vom Sensor 100 bis zur Auswerte- bzw. Verarbeitungselektronik 21 gelegt. Der Kanal 16 ist im einfachsten Fall eine Bohrung durch die Statorblätter 17, die mit dem Pumpenrohr 10 verbunden sind.

In Figur 3 g) kann innerhalb des Pumpenrohrs 10 neben dem eigentlichen drucksensitiven Sensor 100 noch ein Referenz-Drucksensor 103 angebracht sein. Dieser Sensor 103 ist im einfachsten Fall ein IC auf einer elektronischen Platine 22 innerhalb des Pumpengehäuses. Mittels dieses Drucksensors 103 können Schwankungen des Referenzdrucks im hermetisch dichten Pumpengehäuse erfasst werden. Die Druckschwankungen können zur Kompensation von Messfehlern des eigentlichen Sensors 100 verwendet werden.

Figur 4 zeigt eine vereinfachte Darstellung der Anbindung der Sensoren 100 an eine Verarbeitungseinrichtung. Innerhalb des Pumpenrohres 10 können über die elektrischen Verbindungen 100a, 100b, 201 die Signale einzelner Sensoren 100 auf einer Pumpenelektronik 300 zusammengeführt und verarbeitet werden. Dabei können die Umverdrahtungsplatinen 300 rein passiv sein oder bereits eine Vorverarbeitung enthalten.

Das Signal des Drucksensors kann für die Signalaufnahme verstärkt und gefiltert werden. Die Filter können jeweils vor und/oder hinter dem Verstärker angebracht sein. Auch kann der Verstärker als aktives Filterelement ausgeführt sein. Das verstärkte Signal kann von einem Analog-Digital-Wandler (ADC) digitalisiert werden. Nach der Digitalisierung kann der Signalverlauf gespeichert und bei Bedarf weiter gefiltert werden. Eine digitale Filterung beansprucht im Vergleich zur diskreten Filterung keinen Platz in der Pumpe. Die Filter sollen Störungen aus dem Nutzsignal entfernen. Störungen können ihre Quelle außerhalb der Pumpe haben wie Beispielsweise EMV oder Erschütterungen. Eine Störung die von der Pumpe selbst ausgeht ist zum Beispiel das magnetische Streufeld des Magneten im Pumpenrotor. Störungen wie EMV können über eine Frequenzselektion gefiltert werden. Ist mehr über das Störsignal bekannt, dann kann die Störung über ein Korrelationsfilter herausgefiltert werden. Das Korrelationsfilter kann in der Ablaufsteuerung oder als aktives Filterelement ausgeführt werden. Die Störeinflüsse können auch über einen Dummy-Drucksensor aufgenommen werden. Der Dummy-Drucksensor sollte genauso aufgebaut sein wie der Drucksensor, mit dem Unterschied, dass er eine andere Druckabhängigkeit als der Drucksensor aufweist. Die Sensitivität auf Störungen sollte vergleichbar sein. Durch einen Vergleich des Dummy-Drucksensorsignals und des Drucksensorsignals lassen sich die Störeinflüsse aufheben. Vergleichbar verhält es sich mit den Störungen durch das Streufeld des Pumpenrotormagneten. Da die Position und Geschwindigkeit des Rotormagneten dem Motortreiber bekannt ist, kann dieses Signal ebenfalls herausgerechnet werden.

Eine weitere Form von Störungen entsteht bei Vibration oder Beschleinigung der Pumpe. Dieser Störeinfluss kann mit einem Beschleunigungssensor, einem Dummy-Drucksensor oder einem differentiellen Drucksensorpaar gemessen und anschließend gefiltert werden. Werden mehrere Drucksensoren aufgenommen, dann können gemeinsame Elemente der Signalkette mittels eines Multiplexers an die verschiedenen Sensoren angeschlossen werden. Die Position des Multiplexers ist hierbei variabel und kann zum Beispiel nach dem ersten Filter sein.

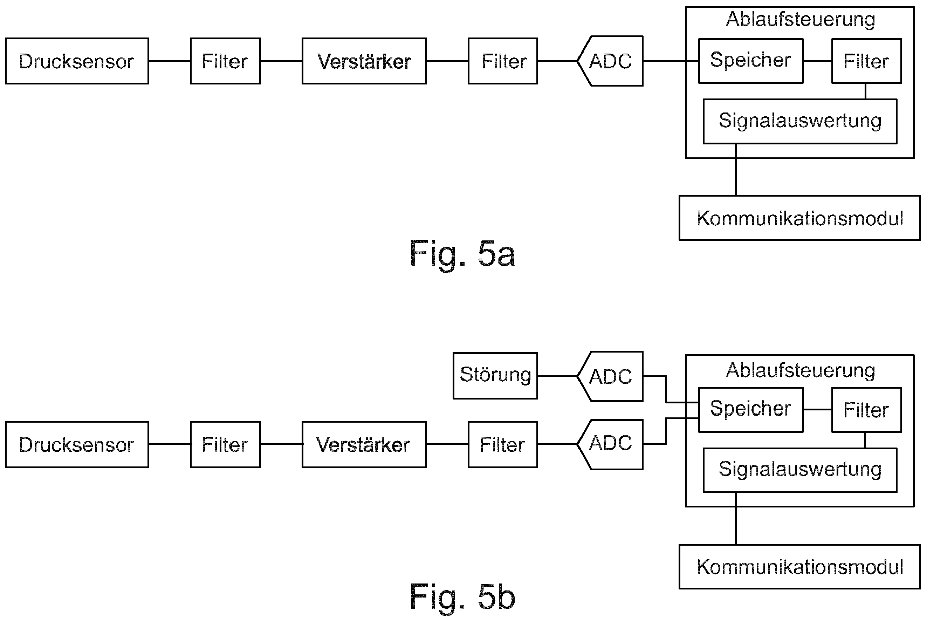

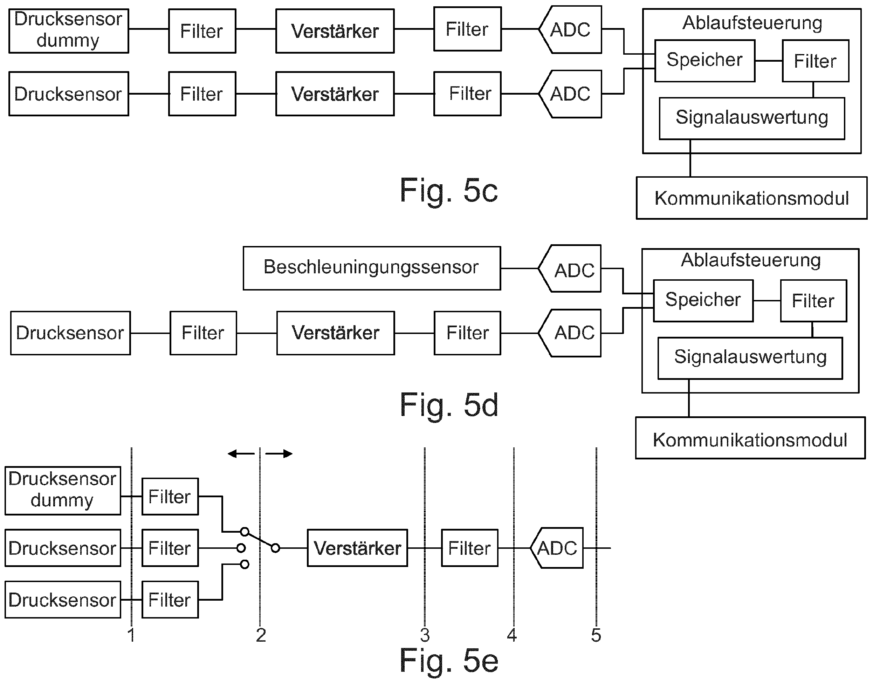

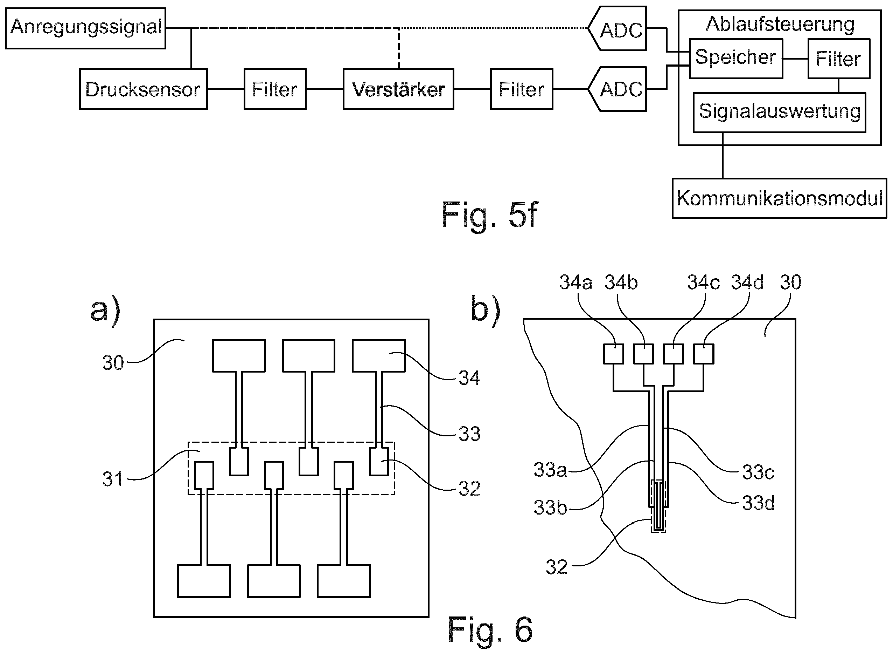

Figur 5 a) zeigt eine Signalkette vom Drucksensor ausgehend bestehend aus Drucktransducer, Filter, Verstärker, ADC, Ablaufsteuerung mit Speicher und Kommunikationsmodul. Die Filter können jeweils weggelassen oder mit angrenzenden Modulen, zum Beispiel dem Verstärker, kombiniert werden.
Figur 5 b) zeigt eine Signalkette ausgehend vom Drucksensor inklusive Abtastung und Filterung der Störeinflüsse.
Figur 5 c) zeigt eine Signalkette ausgehend vom Drucksensor mit Drucksensor-Dummy zur Störunterdrückung.
Figur 5 d) zeigt eine Signalkette mit Drucksensor und Beschleunigungssensor zur Unterdrückung von Schock und Vibration.
In Figur 5 e) kann durch Einsatz eines Multiplexers die Anzahl der Komponenten reduziert werden. Der Multiplexer kann an den Positionen 1 bis 5 positioniert werden.
Figur 5 f) zeigt eine Anregung des Drucksensors mit einem Anregungssignal. Das Anregungssignal kann zusätzlich mit dem Verstärker oder der Ablaufsteuerung verbunden sein. Dies ermöglicht eine sehr selektive Frequenz- oder Korrelationsfilterung.

Zur Temperaturkompensation der Sensoren werden üblicherweise Brückenschaltungen, insbesondere aus vier Sensoren, angewandt. Dies ist jedoch nur erforderlich wenn die störenden Einflüsse das Auflösungsvermögen oder die gewünschte Langzeitstabilität negativ beeinflussen. Insbesondere kommen Halb- und Vollbrücken-Schaltungen zum Einsatz. Figur 6 a) zeigt eine Draufsicht auf einen Sensorbereich 30 mit sechs Sensorstrukturen 32, 33, 34. Figur 6 b) zeigt eine schematische Darstellung einer Sensorstruktur 32, 33, 34 bestehend aus vier zu einer Vollbrücke verschalteten Sensoren. Die Sensoren der Sensorstrukturen 32, 33, 34 sind in einem Bereich 31 maximaler Verformung innerhalb des Sensorbereichs 30 angeordnet. In jeder Sensorstruktur 32, 33, 34 sind vier Sensoren zu einer Vollbrücken-Schaltung 32 (Messbrücke) zur Temperaturkompensation der Sensoren zusammengeschaltet. Jede Vollbrücke 32 weist vier Bond-Pads zur elektrischen Anbindung an eine Verarbeitungseinrichtung auf.

## Patentansprüche

1. Bauteil zum Führen eines Fluids mit einem Sensor, wobei das Bauteil eine innere und eine äußere Wandung umfasst, wobei die innere Wandung zum Führen des Fluids ausgebildet ist, die äußere Wandung das Bauteil nach außen abschließt und zwischen der inneren und der äußeren Wandung ein Wandbereich ausgebildet ist,
**dadurch gekennzeichnet, dass**
der Sensor ein elektromechanisches Sensorelement aufweist und im Wandbereich an der inneren Wandung angeordnet ist, wobei
der Sensor eingerichtet ist, mittels des Sensorelements einen Grad einer Verformung der inneren Wandung im Bereich des Sensors zu messen und als elektrisches Signal auszugeben, wobei
das elektromechanische Sensorelement eine Länge und/oder eine Breite von ≤ 50 µm aufweist.

2. Bauteil nach dem vorhergehenden Anspruch, **dadurch gekennzeichnet, dass** der Sensor zwei elektrische Kontakte aufweist, und das Sensorelement ein nanostrukturelles Dehnelement ist, welches stoffschlüssig auf die Kontakte aufgebracht ist, sodass die Kontakte über das Sensorelement elektrisch miteinander kontaktiert sind, wobei die elektrischen Kontakte derart auf die innere Wandung aufgebracht sind, dass sich das Sensorelement bei Verformung der inneren Wandung im Bereich des Sensors mit der inneren Wandung verformt, sodass zwischen den elektrischen Kontakten das elektrische Signal messbar ist.

3. Bauteil nach dem vorhergehenden Anspruch, **dadurch gekennzeichnet, dass** im Wandbereich an der inneren Wandung eine Vielzahl von Sensoren angeordnet ist, insbesondere dass vier Sensoren angeordnet sind, welche zu einer Voll- oder Halbbrücken-Schaltung miteinander verschaltet sind, um eine Temperaturabhängigkeit der Sensoren zu reduzieren.

4. Bauteil nach einem der vorhergehenden Ansprüche, **dadurch gekennzeichnet, dass** zwischen der inneren Wandung und den elektrischen Kontakten des Sensors eine Isolationsschicht angeordnet ist, welche stoffschlüssig mit der inneren Wandung und den elektrischen Kontakten verbunden ist.

5. Bauteil nach einem der vorhergehenden Ansprüche, **dadurch gekennzeichnet, dass** das elektrische Signal eine Änderung eines elektrischen Widerstands des Sensors beinhaltet.

6. Bauteil nach einem der vorhergehenden Ansprüche, **dadurch gekennzeichnet, dass** das Sensorelement eine Länge und/oder eine Breite von ≤ 15µm, insbesondere ≤ 10 µm, insbesondere ≤ 3 µm, und/oder eine Dicke von ≤ 50 µm, insbesondere ≤ 15µm, insbesondere ≤ 10 µm, insbesondere ≤ 3 µm aufweist.

7. Bauteil nach einem der Ansprüche 2 bis 6, **dadurch gekennzeichnet, dass** die elektrischen Kontakte derart auf die innere Wandung aufgebracht sind, dass zwischen den Kontakten ein Spalt von ≤ 50 µm, insbesondere ≤ 15µm, insbesondere ≤ 10 µm, insbesondere ≤ 3 µm besteht, wobei der Spalt in einer Querrichtung vollständig von dem Sensorelement überdeckt wird.

8. Bauteil nach einem der vorhergehenden Ansprüche, **dadurch gekennzeichnet, dass** der Sensor in einem abgedünnten Bereich der inneren Wandung angeordnet ist.

9. Bauteil nach einem der vorhergehenden Ansprüche, **dadurch gekennzeichnet, dass** in dem Wandbereich elektrische Verbindungselemente angeordnet sind, über welche der Sensor mit einer Verarbeitungseinrichtung verbunden ist.

10. Bauteil nach dem vorhergehenden Anspruch, **dadurch gekennzeichnet, dass** die Verbindungselemente eine Leiterplatte, wobei ein oder mehrere Sensoren direkt über die elektrischen Kontakte oder über Bonddrähte mit der Leiterplatte verbunden sind, oder einzeln isolierte Leitungen, insbesondere Teflon-isolierte Leitungen, umfassen.

11. Bauteil nach dem vorhergehenden Anspruch, **dadurch gekennzeichnet, dass** auf der Leiterplatte elektrische Bauelemente zur Vorverarbeitung der elektrischen Signale, insbesondere zur Vorverstärkung angeordnet sind.

12. Verfahren zur Herstellung eines Bauteils zum Führen eines Fluids mit einem Sensor, umfassend:
Ausbilden einer inneren und einer äußeren Wandung des Bauteils, wobei die innere Wandung zum Führen des Fluids ausgebildet ist und die äußere Wandung ausgebildet ist, das Bauteil nach außen abzuschließen, und die innere und die äußere Wandung derart zusammenfügbar sind, dass sich zwischen der inneren und der äußeren Wandung ein Wandbereich ergibt,
Anordnen eines Sensors mit einem elektromechanischen Sensorelement im Wandbereich an der inneren Wandung, wobei der Sensor eingerichtet ist, mittels des Sensorelements einen Grad einer Verformung der inneren Wandung im Bereich des Sensors zu messen und als elektrisches Signal auszugeben, wobei das elektromechanische Sensorelement eine Länge und/oder eine Breite von ≤ 50 µm aufweist, und
Zusammenfügen der inneren und äußeren Wandung.

13. Verfahren nach dem vorhergehenden Anspruch, **dadurch gekennzeichnet, dass** vor dem Anordnen des Sensors eine Isolationsschicht im Wandbereich auf die innere Wandung aufgebracht wird.

14. Verfahren nach einem der zwei vorhergehenden Ansprüche, **dadurch gekennzeichnet, dass** das Anordnen des Sensors umfasst:
Aufbringen von zwei elektrischen Kontakten im Wandbereich auf die innere Wandung oder auf die Isolationsschicht zum Abnehmen des elektrischen Signals,
Aufbringen des Sensorelements auf die elektrischen Kontakte.

15. Verfahren nach dem vorhergehenden Anspruch, **dadurch gekennzeichnet, dass** das Sensorelement mittels Nano-3D-Drucks, Aufsputterns oder mittels eines Ätzverfahrens auf die Kontakte aufgebracht wird.
